# EUROPEAN PATENT APPLICATION

(11) **EP 1 760 467 A1**
(43) Date of publication of application: **07.03.2007**
(21) Application number: 05019103.0
(22) Date of filing: 02.09.2005
(51) Int. Cl.: G01N 33/58, G01N 21/35, A61K 9/51, A61K 49/04

(54) **Optically fluorescent nanoparticles**

(71) Applicant: Schering AG, 13342 Berlin (DE)
(72) Inventor: Fischer, Katrin Claudia, 10245 Berlin (DE); General, Sascha, 10178 Berlin (DE); Licha, Kai, 14612 Falkensee (DE)
(74) Representative: Kilger, Ute

(57) **Abstract**

The present invention refers to nanoparticles having optically fluorescent activity. In more detail, the invention refers to a nanoparticle matrix comprising a co-aggregate of at least one charged polyelectrolyte and at least one oppositely charged active agent, wherein the active agent is a hydrophilic optically fluorescent agent, and the invention further refers to a nanoparticle comprising said nanoparticle matrix. Optionally, the nanoparticle is surface modified. The invention also refers to a method for preparing said nanoparticle, and to a method of surface modification. Furthermore, the invention refers to uses of said nanoparticle *in vitro* and *in vivo,* and to methods for *in vitro* and *in vivo* diagnosis.

## Description

### OPTICALLY FLUORESCENT NANOPARTICLES

The present invention refers to nanoparticles having optically fluorescent activity. In more detail, the present invention refers to a nanoparticle matrix comprising a co-aggregate of a polyelectrolyte and a hydrophilic optically fluorescent agent, and to a nanoparticle comprising said nanoparticle matrix. Optionally, the nanoparticle is surface modified. The present invention also refers to a method for preparing said nanoparticle and to a method of surface modification. The present invention further refers to uses of said nanoparticle *in vitro* and *in vivo,* and to methods for *in vitro* and *in vivo* diagnosis.

### Background of the invention

For diagnostic purposes, optically fluorescent dyes are particularly useful since low-energy fluorescent light is biologically safe compared to, for example, high-energy radioactive radiation. Today, optically fluorescent dyes are widely used in *in vitro* diagnostic methods whereby the exposure of laboratory stuff to potentially harmful radiation is reduced. In *in vivo* imaging methods, however, biological safety is even more significant and thus, attempts have been made to take advantage of optically fluorescent dyes in such diagnostic methods as well.

Fluorescence imaging uses, as a contrast agent, a substance that emits fluorescence upon exposure to an excitation light having a specific wavelength. Thus, a patient or a part of the body is exposed to an excitation light from outside the body, and the fluorescence emitted from the fluorescent contrast agent in the body is detected.

Fluorescent dyes such as fluorescein, fluorescamine and riboflavin emit in a region of visible light of about 400 to 600 nm. In this region, the light transmission through living tissue is very low, so that a detection in deep parts of the body is nearly impossible.

US 2004/213740 discloses highly hydrophilic indole and benzoindole derivatives that absorb and emit in the visible region of light. These compounds are useful for physiological and organ function monitoring, in particular for optical diagnosis of renal and cardiac diseases and for estimation of blood volume *in vivo.*

Imaging of deeper tissue, however, requires optically fluorescent dyes emitting in the near infrared (NIR; 600-1000 nm) [Intes and Chance (2005)]. This is due to the fact that haemoglobin and water, the major absorbers of visible and infrared light, respectively, have their lowest absorption coefficient in the NIR region in the range of 650-900 nm. NIR light has been shown to travel at least though 10 cm of breast tissue and 4 cm of skull/brain tissue or deep musculature using microwatt laser sources (Food and Drug Administration, FDA, class 1¹⁰) [Weissleder (2001a)].

WO 00/16810 discloses a NIR fluorescent contrast agent comprising a compound having three or more sulfonic acid groups in a molecule, and a method of fluorescence imaging comprising introducing the NIR fluorescent contrast agent into a living body, exposing the body to an excitation light and detecting NIR fluorescence from the contrast agent. According to WO 00/16810, the transmission of light provided by the disclosed NIR fluorescent dye through biological tissues is superior. Thus, detections in the deep part of a living body have become available. In addition, the inventive contrast agent is superior both in water solubility and low toxicity, and therefore, it can be used safely. One specific compound which is comprised by the NIR fluorescent contrast agent disclosed in WO 00/16810 is tetrasulfonated indotricarbocyanine.

Such NIR dyes have also turned out as particularly useful for molecular imaging. The term "molecular imaging" can be broadly defined as the *in vivo* characterization and measurement of biologic processes at the cellular and molecular level. In contradistinction to conventional diagnostic imaging, it sets forth to probe the molecular abnormalities that are the basis of disease rather than to image the end effects of these molecular alterations [Weissleder (2001b)].

However, high performance of any *in vivo* imaging method requires optimized discrimination of pathologically altered tissue, e.g. tumour or inflammatory tissue, and surrounded unaltered tissue. Sensitivity and reliability of the method depends on the ratio of a true positive signal and a negative or false positive background signal. This ratio is not only affected by transmission properties of the fluorescent dye, but also by the dye's distribution within the tissue. Therefore, besides the development of fluorescent dyes having improved transmission properties, another challenge is to maximize the enrichment of fluorescent dyes in the pathologically altered tissue.

One approach to label cells and tissues selectively having undergone pathologic changes is the targeting of cell surface structures that are specifically or at least predominantly expressed in and exposed on the tissue of interest, e.g. by antibodies. However, production and NIR dye labelling of such antibodies is often laborious. Moreover, recombinant antibodies as commonly used are still associated with the risk of adverse immunogenic side-effects when applied systemically.

Alternatively, specifically expressed cell surface structures, e.g. receptors, can be targeted by small molecular targeting probes, e.g. ligands or ligand mimetics. One such example is folic acid conjugated to a NIR dye targeting the folate receptor overexpressed on many tumour types [Tung *et al.* (2002)].

Interestingly, it has been shown that pathologically altered tissues can be targeted by particles, preferentially in the nanometer scale, also referred to as nanoparticles. If such systems are used to target tumour tissue, for example, possible side effects of therapeutic agents may be reduced [Dass 2000]. Predominantly, the mechanism is due to the more loose and porous structure of solid tumour tissues resulting in enhanced accessibility compared to healthy tissues, which mechanism is known as passive targeting [Liotta *et al.* (1976)]. Thus, nanoparticles offer the opportunity to stain selectively pathologically altered tissues, using the commonly known "enhanced permeability and retention effect" (EPR effect). Modifying the surface of these nanoparticulate complexes, e.g. with target specific molecules, combines passive and active targeting effects and results in a more specific accumulation in the targeted tissue.

Particulate systems in the nanometer scale that have been widely used in the field of medicine are liposomes composed of non-toxic natural lipids, predominantly phospholipids. A more recent variant thereof are polymersomes prepared from synthetic polymers. Liposomes and polymersomes are vesicles formed spontaneously in aqueous media due to the amphiphilic character of their components. Hydrophilic molecules are entrapped in the aqueous interior, but hydrophobic molecules can be hosted in the membrane. However, the capacity of loading is limited because of the location either in the membrane or in the core. Hydrophilic coating of the vesicles' surfaces may extent their circulation half-life and therefore their localisation in the tissue ("stealth liposomes"). A more sophisticated approach are vesicles capable of targeting selectively cell surface molecules on malignant cells. For that purpose, targeting moieties such as antibodies are attached to the surface of the vesicles. However, a low payload of fluorescence dyes combined with a too rapid release from such vesicles does not allow sufficient concentration in the tissue to be investigated [Derycke and Witte (2004)].

Alternatively, biodegradable polymers such as polylactic acid (PLA) or polylactic-co-glycolic acid (PLGA) can be used for the preparation of nanoparticles. In that case, the fluorescent dye, e.g. indocyanine green, is released in the course of biodegradation of the matrix forming polymer [Saxena *et al.* (2004a); Saxena *et al.* (2004b)]. However, this process is running too slowly to provide sufficient concentration of the fluorescent dye at a given time. Moreover, as also disclosed in WO 2004/064751, the loading capacity with hydrophilic fluorescent dyes is particularly low.

US 2005/0019265, and similarly US 2005/0003016, discloses polymersomes in the range of about 50 nm to about 50 µm comprising a plurality of amphiphilic co-polymers and at least one visible- or near infrared-emissive agent that is dispersed within the polymersome membrane. In addition to amphiphilic block-co-polymers, the polymersomes can comprise hydrophobic and hydrophilic polymers. In some embodiments, the multiblock polymers can be crosslinked and the fluorophores are embedded therein. The polymersomes are considered for use in diagnostic or imaging methods *in vitro* and *in vivo.* Optionally, the polymersome surface is modified with biological moieties to improve selectivity. It is described that the thick membranes by which the polymersomes are characterised are of advantage since they allow the incorporation of large fluorophores which can not be introduced in natural membranes composed of phospholipids. However, the optically fluorescent agents should be hydrophobic to render the dye substantially soluble within the polymersome membrane. An application of polymersomes in diagnostic *in vitro* or *in vivo* methods without the requirement of actively targeting moieties is not explicitly mentioned in US 2005/0019265.

From EP 0 454 044, and also from CA 2041093, the preparation of colloidal nanoparticles from polyelectrolyte complexes composed of polycationic and polyanionic compounds and at least one active agent is known. Amongst other mechanisms described by which the active agent can be introduced into the nanoparticles, the active agent can directly act as a polyelectrolyte complex partner. In that case, however, the active agent requires at least one charge or functional group which can be polarised. The use of optically fluorescent agents is not mentioned in EP 0 454 044.

WO 2004/096998 discloses a nanoparticle delivery system capable of targeting tumour vasculature and delivering anti-angiogenic compounds. The nanoparticle system comprises a water-based core and a water-based corona surrounding the core. The core comprises at least one polyanionic polymer, and the corona comprises at least one polycationic polymer and a targeting ligand which is cross-linked or conjugated to a polymer. In addition to selective targeting, the nanoparticles also may perform non-invasive imaging using bioluminescence and/or magnetic resonance (MR) imaging. A contrast agent emitting in the NIR is not explicitly mentioned.

Imaging probes combining MR and optically fluorescent detection have been developed in order to obtain more detailed anatomic and molecular information in living organisms [Josephson *et al.* (2002)]. By MR imaging, the nanoparticulate probes are localized while optically fluorescent imaging provides information about molecular properties of the localised tissue.

DE 102 36 409 discloses microcapsules having in a layer-by-layer structure, wherein at least one layer comprises fluorescent dyes. Colloids in a mono-dispersion are coated with polyelectrolytes. Dyes of different fluorescent colours are comprised by the polyelectrolytes with a covalent bonding wherein fluorescein isothiocyanate and tetramethyl rhodamine isothiocyanant are mentioned explicitly. Barriers are between the layers to block undesired reactions. The capsule coding is read by the variation in the excitation and emission wavelength.

WO 2004/108902 discloses fluorescent silicon nanoparticles having a biocompatible coating which can be used as imaging probes both *in vitro* and *in vivo* optical molecular imaging. It is preferred that the nanoparticles have NIR fluorescence capability.

The incorporation of complexes prepared from an active agent and cyclodextrin into polymer nanoparticles has also been reported [Duchene *et al.* (1999); Boudad *et al.* (2001)]. Cyclodextrin was used with the purpose to overcome weak solubility of an active agent under physiological conditions after parental or oral application. Beta-Cyclodextrin is widely used in oral pharmaceutical compositions as a solubilising agent, as a stabilising agent in preparations of vitamins and for improvement of taste and odour. The property of cyclodextrin as a solubilising agent is based on the formation of an inclusion complex wherein a hydrophobic active agent is entrapped into the hydrophobic core. In some cases, the inclusion is accompanied by enhanced chemical and physical stability of the active agent. Amongst the cyclodextin derivatives, hydroxy-beta-cyclodextrin has already been registered as an ingredient of an infusion solution (Sempera^{®}). Furthermore, cationic modified cyclodextins in particulate compositions have been described as valuable alternatives in gene transfection technology [Bellocq *et al.* (2003); Pun *et al.* (2004); WO 03/072367; WO 02/49676]. When cyclodextrin inclusion complexes containing hydrocortisol as the active agent were incorporated into so-called solid lipid nanoparticles (SLN), the loading capacity of the active agent was enhanced. Correspondingly, the release of hydrocorisol was reduced compared to preparations without cyclodextrin [Cavalli *et al.* 1999].

In order to use fluorescent imaging as a biologically safe alternative for contrasting tissue, improved systems for delivery of the NIR dye to the target tissue has to be developed. Obstacles such as immunogenic effects of antibodies, a low payload of conventional polymeric nanoparticles and their slow release behaviour based on polymer degradation show that there is still a potential for innovative solutions to overcome these problems.

In consequence, there is still a need in the art of biologically safe, selective and sensitive tools for use in diagnostic methods and for monitoring therapeutic interventions.

Thus, the object of the present invention is to provide appropriate tools and methods using the same.

### Summary of the present invention

The object of the present invention is solved by a nanoparticle matrix comprising a co-aggregate of at least one charged polyelectrolyte and at least one opposite charged active agent, wherein the active agent is a hydrophilic optically fluorescent agent.

In one embodiment, the ratio of total polyelectrolyte charge and total optically fluorescent agent charge results in a charge surplus.

In one embodiment, the optically fluorescent agent is a visible- or NIR-emissive compound.

In one embodiment, the optically fluorescent agent is a NIR-emissive compound.

In one embodiment, the optically fluorescent agent comprises a planar aromatic and highly conjugated system.

In one embodiment, the optically fluorescent agent shows a bathochromic shift in UV-Vis absorption spectrum during co-aggregate formation.

In a preferred embodiment, the optically fluorescent agent shows a bathochromic shift in UV-Vis absorption spectrum during co-aggregate formation and a hypsochromic shift during disaggregation.

In a particularly preferred embodiment, the bathochromic and/or hypsochromic shift is characterised by a Δ_{wavelength} which is in the range of about 20 nm to about 80 nm.

In a more particularly preferred embodiment, the bathochromic and/or hysochromic is characterised by a Δ_{wavelength} which is in the range of about 30 nm to about 50 nm.

In a more particularly preferred embodiment, the bathochromic and/or hysochromic shift is characterised by a Δ_{wavelength} of about 40 nm.

In one embodiment, the optically fluorescent agent shows a decrease in absorption intensity during co-aggregate formation due to quenching effects.

In a preferred embodiment, the optically fluorescent agent shows a decrease in absorption intensity during co-aggregate formation and an increase in absorption intensity during disaggregation due to the reversibility of quenching effects when dye molecules are separated from the complex.

In one embodiment, the polyelectrolyte is cationic and the optically fluorescent agent is anionic.

In one embodiment, the ratio of total cationic polyelectrolyte charge and total anionic optically fluorescent agent charge is in the range of about 1.5:1 to about 6:1.

In a preferred embodiment, the ratio of total cationic polyelectrolyte charge and total anionic optically fluorescent agent charge is in the range of about 1.5:1 1 to about 3:1.

In a particularly preferred embodiment the ratio of total cationic polyelectrolyte charge and total anionic optically fluorescent agent charge is about 1.5:1.

In one embodiment, the cationic polyelectrolyte is selected from the group consisting of polyethyleneimine (PEI) and derivatives such as polyethylene[113]-b-polyethyleneimine[30] (PEG[113]-b-PEI[30]); polyvinyl derivatives such as polyvinylamine and polyvinylpyridin; polyarginine, polyhistidine; polylysine, lysine octadecyl ester; polyguanidine and poly(methylene-co-guanidine); protamines such as protamine sulfate; polyallylamine, polydiallyldimethylamine; polymethacrylates such as Eudragit E, poly(dimethylaminopropyl-methacrylamide) [P(DMAPMAM)] or poly(dimethyl-aminoethyl-methacrylate) [P(DMAEMA]; spermine, spermidine and their polymers polyspermine and polyspermidine; quaternised polyamide, poly(dimethyl-aminoethyl-aspartamid) [PDAA]; modified silicones; polyphosphazene; proteins such as histones; modified starch, modified gelatine, modified cellulose such as aminated celluloseethers; aminated dextrans, aminated cyclodextrins, aminated pectines; chitosan; and salts and derivatives thereof.

In a preferred embodiment, the cationic polyelectrolyte is polyethyleneimine.

In one embodiment, the optically fluorescent agent comprises a surplus of negatively charged groups selected from the group consisting of sulfonate and phosphate.

In one embodiment, the optically fluorescent agent is a polymethine dye, preferably a cyanine dye.

In a preferred embodiment, the optically fluorescent agent is a indotricarbocyanine dye or a indodicarbocyanine dye.

In a more preferred embodiment, the optically fluorescent agent is a tetrasulfonated tricarbocyanine dye.

In a particularly preferred embodiment, the optically fluorescent agent is trisodium-3,3-dimethyl-2-{4-methyl-7-[3,3-dimethyl-5-sulfonato-1-(2-sulfonatoethyl)-3H-indolium-2-yl]hepta-2,4,6-trien-1-ylidene}-1-(2-sulfonatoethyl)-2,3-dihydro-1H-indole-5-sulfonate, inner salt, abbreviated to TITCC.

Alternatively, in an particularly preferred embodiment, the optically fluorescent agent is disodium-3,3-dimethyl-2-{7-[3,3-dimethyl-5-sulfonato-1-(2-sulfonatoethyl)-3H-indolium-2-yl]-hepta-2,4,6-trien-1-ylidene}-1-(2-sulfbnatoethyl)-2,3-dihydro-1H-indole-5-carboxylic acid-(11-carboxyundecyl)-amide, abbreviated to Dye-12-aminododecanoic acid conjugate.

In one embodiment, the matrix additionally comprises at least one auxiliary electrolyte.

In a preferred embodiment, the auxiliary electrolyte is selected from the group consisting of modified cyclodextrins, chelating agents, dendrimers and crown ethers.

In a more preferred embodiment, the auxiliary electrolyte is charged opposite to the polyelectrolyte.

In a more preferred embodiment, the modified cyclodextrin is an anionic cyclodextrin selected from the group consisting of phosphated, sulfated, carboxymethylated, and succinylated cyclodextrin.

In a most preferred embodiment, the anionic cyclodextrin is heptakis-(2,3-dimethyl-6-sulfato)-beta-cyclodextrin or heptakis-(2,6-diacetyl-6-sulfato)-beta-cyclodextrin.

Alternatively, in a most preferred embodiment, the anionic cyclodextrin is beta-cyclodextrin phosphate.

The object of the present invention is further solved by a nanoparticle comprising the nanoparticle matrix according to the present invention.

In one embodiment, the nanoparticle is non-vesicular.

In one embodiment, the nanoparticle size is in the range of about 10 nm to about 1.2 µm.

In a preferred embodiment, the nanoparticle size is in the range of about 10 nm to about 500 nm.

In a particularly preferred embodiment, the nanoparticle size is in the range of about 10 nm to about 300 nm.

In one embodiment, the nanoparticle comprises at least one surface modifying agent.

In a preferred embodiment, the surface modifying agent charged is opposite to the nanoparticle's surface charge.

In a particularly preferred embodiment the surface modifying agent is polyethyleneglycol[110]-b-glutamic acid[10] (PEG[110]-b-Glu[10]).

In an alternatively preferred embodiment, the surface modifying agent is selected from the group consisting of NADP, AMP, cAMP and ADP and salts thereof.

In one embodiment, the nanoparticle comprises a targeting structure.

In a preferred embodiment, the targeting structure is a passively targeting structure.

In an alternatively preferred embodiment, the targeting structure is an actively targeting structure.

In a particularly preferred embodiment, the actively targeting structure is a region of an antibody, of a non-antibody ligand, of an aptamer, or of fragments thereof.

In one embodiment, the nanoparticle comprises a therapeutically active agent.

In a preferred embodiment, the therapeutically active agent is selected from the group consisting of anti-proliferating agents, anti-inflammatory agents, and dyes for photodynamic therapy.

The object of the present invention is further solved by a method of preparation of a nanoparticle according to the present invention comprising the following steps:
(a) contacting at least one polyelectrolyte and at least one optically fluorescent agent;
(b) co-aggregating the polyelectrolyte and the optically fluorescent agent under UV protection;
(c) yielding the co-aggregation product.

In one embodiment, the method of preparation additionally comprises the following step:
(b') modifying the co-aggregation product's surface.

In one embodiment, the co-aggregation in step (b) is carried out at about 4°C.

In one embodiment, the co-aggregation in step (b) is carried out at about pH 7.0-9.0.

In a preferred embodiment, the co-aggregation in step (b) is carried out at about pH 7.5-8.5.

In one embodiment, the co-aggregation in step (b) is monitored by running an UV-Vis spectrum.

The object of the present invention is further solved by a method of surface modification of a nanoparticle according to the present invention comprising the step of applying a surface modifying agent according to the present invention onto the nanoparticle's surface.

The object of the present invention is further solved by a use of a nanoparticle according to the present invention *for in vitro* application.

The object of the present invention is further solved by a method for *in vitro* diagnosis using nanoparticles according to the present invention comprising the following steps:
(a) contacting the nanoparticles with a biological sample;
(b) removing non-bound nanoparticles from the biological sample;
(c) detecting nanoparticles bound to the biological sample;
(d) comparing the result obtained in step (c) with a result obtained from a control sample.

In one embodiment of the method for *in vitro* diagnosis, the detection in step (c) is carried out by using a fluorescent microscopic technique.

The object of the present invention is further solved by a use of a nanoparticle according to the present invention for *in vivo* application.

The object of the present invention is further solved by a method for *in vivo* diagnosis using nanoparticles according to the present invention comprising the following steps:
(a) applying the nanoparticles to a subject;
(b) detecting nanoparticles accumulated in the subject.

In one embodiment, the detection in step (b) is carried out by using a CCD (charge coupled device) technique.

The object of the present invention is further solved by a method for *in vivo* localisation of a nanoparticle according to the present invention.

In one embodiment of the method for *in vivo* localisation, the nanoparticle comprising at least one therapeutically active agent.

The object of the present invention is further solved by a use of a nanoparticle according to the present invention for the manufacture of a pharmaceutically acceptable composition.

The object of the present invention is further solved by a pharmaceutically acceptable composition comprising a nanoparticle according to the present invention.

The object of the present invention is further solved by a kit for *in vitro* and/or *in vivo* diagnosis comprising the nanoparticle according to the present invention.

The term "nanoparticle matrix" as used in the present invention refers to a material that forms the basis material of the nanoparticle. As a rule, said matrix has an organised structure.

The term "co-aggregate" as used in the present invention refers to an aggregate requiring at least two different components that interact as aggregation partner. As used herein, the aggregation partners are at least one charged polyelectrolyte and at least one hydrophilic optically fluorescent agent.

The term "polyelectrolyte" refers to a polymer containing multiple ionic groups in its polymer chain and/or substituents. The group of polyelectrolytes comprises polyanions, polykations and polyampholytes. Oligoelectrolytes are explicitly contemplated as well.

If not already mentioned in single cases, the term "polyelectrolyte" further comprises block-co-polymers or random polymers. A combination of a block or random polymer and uncharged compounds is also considered. In addition, coupling of these polymers and copolymers to various sugars or a partially hydrophobic substitution of the derivatives is considered. If PEI is used, this polyelectrolyte is available of various molecular weights, and amongst PEI derivatives, ethoxylated derivatives are preferred.

The terms "polyelectrolyte charge" and "optically fluorescent agent charge" refer to the amount of charges per molecule.

The term "optically fluorescent agent" refers to a compound capable of emitting energy in form of visible or NIR radiation previously absorbed during exposure to, for example, visible light or NIR radiation (excitation).

The term "bathochromic shift" means a shift in absorption at a certain wavelength, in particular at an absorption maximum, towards longer wavelengths. The term "hypsochromic shift", vice versa, means a shift towards shorter wavelengths. A "shift" means an increase or decrease in the wavelength, i.e. a the difference between the wavelength prior to the shift and after the shift. As used herein, the difference is indicated by "Δ_{wavelength} [nm]".

The "decrease in absorption intensity" during co-aggregate formation is due to quenching effects and J-aggregate formation. In the art, such a decrease is also referred to as "hypochromic effect". Vice versa, the "increase in absorption intensity" is also known as "hyperchromic effect".

The "auxiliary electrolyte" is added to support the co-aggregate formation of polyelectrolyte and optically fluorescent agent by interacting with the optically fluorescent agent. This is particularly useful with dyes carrying very few charges, e.g. indocyanine green, since the auxiliary electrolyte provides additionally charges. In this case, the auxiliary electrolyte and the optically fluorescent agent are precipitated together with the polyelectrolyte. When modified cyclodextrins are used as auxiliary electrolytes, they may be selected of α-, β-, γ-, or δ-modfied cyclodextrins.

It is also contemplated by the present invention that the polyelectrolyte or oligoelectrolyte is anionic and the optically fluorescent agent is cationic. In that case, the anionic polyelectrolyte preferably is selected from the group consisting of alginic acid, hyaluronic acid, polyvinylsulfonic acid, polyvinylphosphoric acid, gummi arabicum, pectines, nucleic acids, anionic proteins, lignin, sulfonic acid and anionically modified clyclodextrins.

The term "nanoparticle" refers to a colloidal particle having a size in the nm range. A nanoparticle can describe a vesicular or a non-vesicular nanoparticle. A "vesicular nanoparticle", as used herein, is characterised in that the nanoparticle matrix forms a body of more or less spherical shape enclosing an internal cavity without matrix. The internal cavity is commonly filled with a fluid medium, e.g. a liquid, which is the same as or different from the external medium. A "non-vesicular nanoparticle", as used herein, in contrast, is lacking the internal cavity.

One mechanism of modification of the nanoparticle's surface by a "modifying agent" is based on electrostatic interactions. Preferably, the modifying agent is selected from the group of polymers such as modified polyethylene glycols that cause a sterical or electrostatic stabilisation or both, and shields the nanoparticle from proteins and other components of blood plasma in order to extent half-life in circulation. Electrostatic modification of the nanoparticle's surface can also lead to active targeting and can be in a combination with van der Waals forces, dipol-dipol and other non-covalent interactions. Alternatively, the modifying agent is capable of covalent interactions with functional groups contained in the nanoparticle matrix. PEG[110]-GLU[10] has been shown to increase the plasma half-life when applied onto the surface of a nanoparticle comprising a PEI based nanoparticle matrix. Moreover, the use of NADP sodium salt has been shown to enhance fluorescence intensity.

The term "targeting structure agent" refers to a moiety capable of specifically targeting cells or tissues, e.g. a ligand specifically targeting its corresponding receptor. Those skilled in the art would readily recognise that any targeting structure which enhances uptake or localisation in a given tissue may be an appropriate candidate for targeting. This structure may be part of a targeting molecule such as an antibody or a fragment thereof, a non-antibody ligand or fragment binding to a cellular receptor, e.g. a cell surface receptor, or an aptamer. The structure can be a peptide, a carbohydrate, a protein, a lipid, a nucleoside, a nucleic acid, a polysaccharide, a modified polysaccharide or fragments thereof. In addition, the ligand can be transferrin or folic acid. Optionally, the targeting molecule can be bound to a component of the nanoparticle matrix or of the nanoparticle. Besides whole targeting molecules, the term "targeting structure" also comprises a certain region of a molecule.

"Active targeting" means, that targeting is mediated by, for example, a receptor/ligand interaction. Normally, active targeting depends on the extent of fitting of complementary structures comprised by the interaction partnerts. "Passive targeting", in contrast, is supported by the particular size and nature, e.g. hydrophobicity and charge, of a nanoparticle.

The term "therapeutically active agent" considers all active agents currently known in the art. Active agents useful for gene therapy are also considered by the present invention.

In the method of preparation of a nanoparticle, the polyelectrolyte and the optically fluorescent agent are preferably solved in an aqueous solvent. Preferably, the co-aggregation formation is supported by gentle agitation for about 30 to 60 min, more preferably for about 45 min. The suspension obtained at the end of the co-aggregation step may be concentrated, e.g. by means of a Millipore ultra filtration cell or by vacuum evaporation, in particular if a modification step follows. The co-aggregation product yielded may be obtained by lyophilisation in the presence of any appropriate cryoprotector, preferably mannitol or lactose. In case an auxiliary electrolyte is used, it is added.

In the method *for in vitro* diagnosis, the biological sample is obtainable from a subject, preferably a vertebrate, more preferably a mammal, most preferably a human. The sample is selected from the group consisting of cells, cell lysates, bodily fluid, tissue and tissue homogenates and extracts. The nanoparticles are preferably dispersed in a liquid vehicle.

A "control sample" as used in the method *for in vitro* diagnosis may refer to a sample obtained from a healthy subject. Alternatively, the result obtained may be compared to standardized parameters.

Also in the method for *in vivo* diagnosis, the subject preferably is a vertebrate, more preferably a mammal, most preferably a human. The nanoparticles are preferably dispersed in a pharmaceutically acceptable liquid vehicle, more preferably an aqueous vehicle, most preferably physiologic saline. The application route may be a systemic one, e.g. i.v. infusion, i.v., s.c. or i.m. injection, or can be local. Application routes considered comprise oral, nasal, rectal and vaginal application.

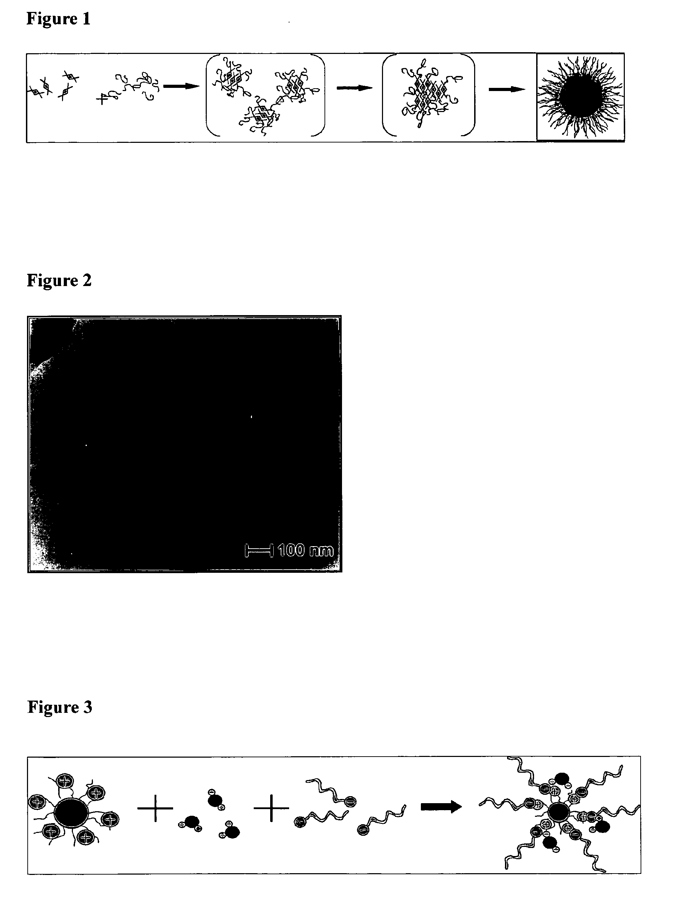

The nanoparticles of the present invention are characterised by a nanoparticle matrix comprising a co-aggregate of a polyelectrolyte and a hydrophilic optically fluorescent agent wherein the latter is directly involved in the nanoparticle matrix formation by acting as a partner of the polyelectrolyte. Under certain conditions, of which the charge ratio of the aggregation partners is essential, precipitation of stable nanoparticles occurs resulting in the formation of J-aggregates due to electrostatical and π-π interaction. A scheme depicting the principal of co-aggregate formation according to the present invention is shown in *Fig. 1.*

Because of the nanoparticles' self stabilisation by their charges, the addition of surfactants is mostly unnecessary. Nevertheless, in certain applications, an additional use of a surfactant may be of further advantage and is considered by the present invention.

J-aggregates are characterised in that the components organises to structures of high order. Different packing types such as the "brick type" or the "sandwich type" are known. J-aggregates of cyanine dyes have been extensively studied because of their interesting optical properties and their possible application to optoelectronic devices [Jelly, E.E. *et al.* (1936); Kobayashi, T. (1996)]. Rousseau *et al.* (2002) studied the influence of different cationic polymers like poly(diallyldemethylammonium chloride) or poly(allylamine hydrochloride) on the formation of J-aggregates with the thiacarbocyanine dye THIATS.

Furthermore, J-aggregates are characterised in that the molecules are at a lower energy level compared to the non-aggregated state. Thus, when the co-aggregate of the present invention forms, a bathochromic shift in absorption of the optically fluorescent agent is observed. In case of TITCC, the shift is from 756 nm to a wavelength in the range of 795 and 810 nm, depending on the exact composition of fluorescent dye and polyelectrolyte. Thus, Δ_{wavelength} is from +39 nm to +54 nm in case of the bathochromic shift, and vice versa, Δ_{wavelength} is from -39 nm to -54 nm in case of the hypsochromic shift.

Moreover, due to the highly organised structure of molecules in J-aggregates, the fluorescent emission of the dyes is quenched, i.e. the emission intensity is decreased compared to the non-aggregated state. Vice versa, disaggregation of a nanoparticle of the present invention is accompanied by an increase in emission intensity.

The nanoparticles of the preferred embodiment of the present invention are thus characterised by (1) a bathochromic shift in absorption and a decrease in emission intensity when co-aggregates are formed, and (2) a hypsochromic shift in absorption and an increase in emission intensity during disaggregation. On the basis of these characteristics, the physical state of a nanoparticle, i.e. co-aggreagated or disaggregated, can be discriminated.

Moreover, since the optically fluorescent dye is part of the nanoparticle's matrix, the dye is highly concentrated within the nanoparticle.

The nanoparticles of the present invention are particularly useful for *in vivo* and *in vitro* diagnosis. After application of nanoparticles of the present invention to a live organism, they particularly enrich in tumour tissue. This is due to passive and active targeting effects. Once located in targeted tissue, the inventive nanoparticles are taken up by tissue cells via endocytosis. Following this cellular incorporation, disaggregation into the nanoparticle's components occurs resulting in "free" optically fluorescent agent. From *in vitro* experiments using blood plasma it has been concluded that disaggregation is supported by changes of ionic concentration and the presence of charged blood compounds, e.g. albumin or salts. Intracellular disaggregation is possibly proceeded by the "proton sponge" effect. Furthermore, disaggregation of the nanoparticle occurs independently of any polymer degradation resulting in rapid release of the optically fluorescent agent and providing a high dye concentration for detection.

Thus, the nanoparticles of the present invention selectively enrich in pathologically altered tissue due to passive and active targeting and, simultaneously, the sensitivity of *in vivo* imaging is enhanced since the fluorescent signal of intracellularly free optically fluorescent dye is increased.

In addition, the nanoparticles of the present invention offer the opportunity to observe the localisation and disaggregation of nanoparticles comprising therapeutically active agents and thus to check the localisation and the delivery of the therapeutically active agents. In this special case, the nanoparticles of the present invention serve both as carriers of the optically fluorescent agent and as drug carriers.

Finally, the outstanding properties of the nanoparticles discussed above can be advantageously used in *in vitro* systems for studying the stability of nanoparticles, in particular the effect of surface modifications. It has been shown that both the co-aggregation components and any surface modifications can greatly influence the nanoparticles' stability and thus their disaggregation profile. Furthermore, such an *in vitro* system can generally be used for designing nanoparticles with respect to the selection of the polyelectrolyte and optically fluorescent agent as well as to the development of nanoparticle surface modifications.

### Detailed description of the invention

The present invention will be outlined below in more detail by presenting particular embodiments and examples, and making reference to the attached figures.

### Brief description of the Figures

- *Fig. 1*: is a scheme showing the aggregation of optically fluorescent agent and polyelectrolyte resulting in precipitation of nanoparticles.
In the figure, the optically fluorescent agent is represented by the diamond-shaped components.
- *Fig. 2*: is a transmission electron microscope (TEM) picture showing nanoparticles composed of PEI and TITCC.
- *Fig. 3*: is a scheme showing a surface modification of PEI/TITCC nanoparticles with NADP disodium salt plus PEG[110]-b-GLU[10].
In the figure, a positively charged nanoparticle without surface modification (on the left) together with the anionic component of NADP disodium salt (black spheres having negative charges) and negatively charged PEG[110]-b-GLU[10] (spheres having tails) form a surface modified nanoparticle (on the right).

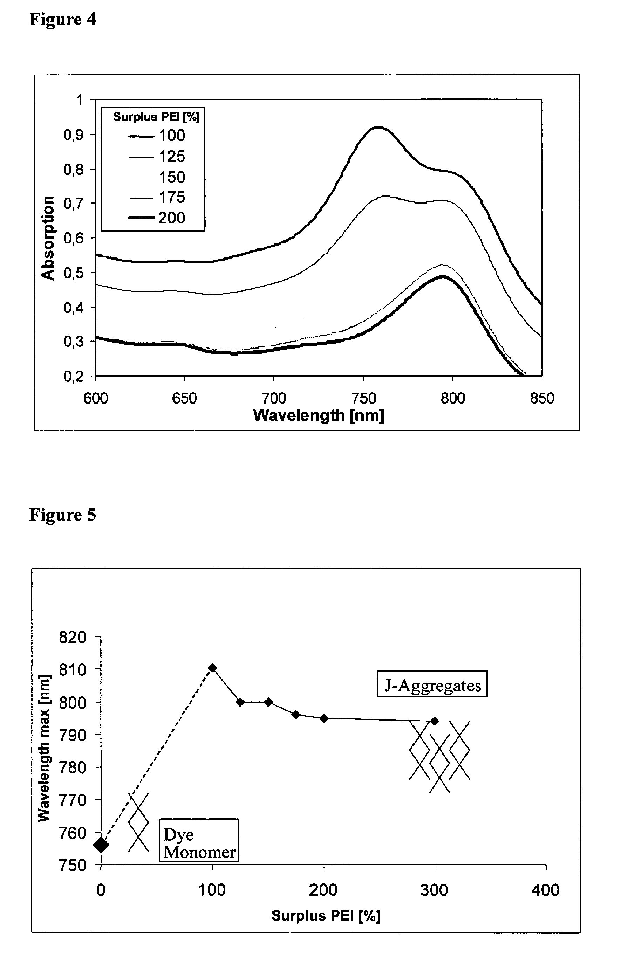

- *Fig. 4*: shows UV-Vis spectra of the shift in the absorption during the formation of PEI/TITCC nanoparticles.
- *Fig. 5*: shows the absorption maxima of PEI/TITCC nanoparticles as a function of the dye polymer ratio.

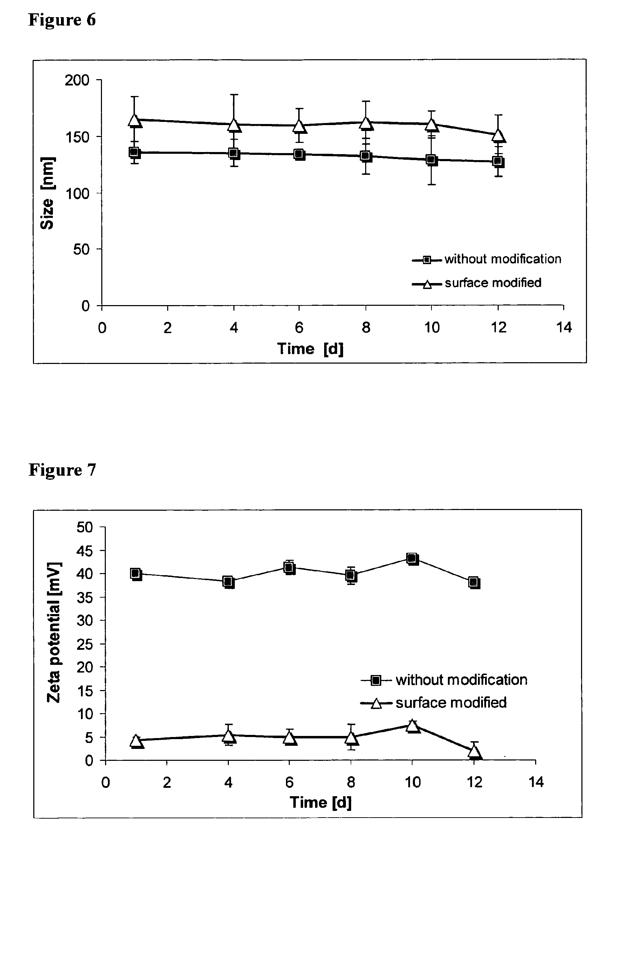

- *Fig. 6*: is a graph showing stability data of the size of PEI/TITCC nanoparticles with or without surface modification as illustrated in *Fig. 3* after incubation in an incubation shaker at 37°C.
- *Fig.* 7: is a graph showing stability data of the zeta potential of PEI/TITCC nanoparticles with and without surface modification as illustrated in *Fig.* 3.

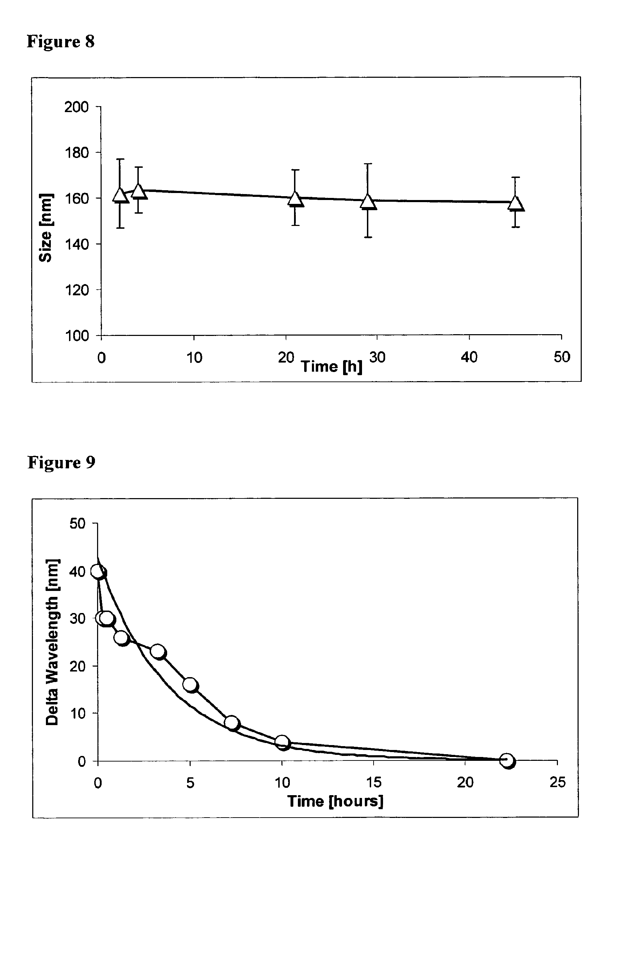

- *Fig. 8*: is a graph showing stability data of PEI/TITCC nanoparticles in plasma with surface modification as illustrated in *Fig.* 3.
- *Fig. 9*: is a graph showing the time-course of disaggregation of PEI/TITCC nanoparticles in plasma with surface modification as illustrated in *Fig.* 3.

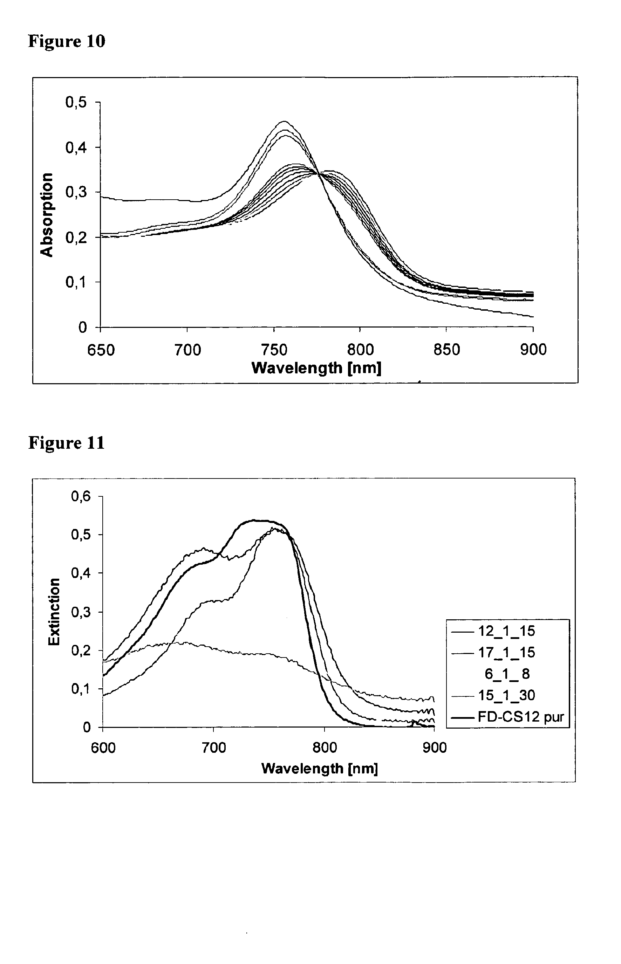

- *Fig. 10*: shows UV-Vis spectra of the disaggregation process of PEG[113]-PEI[30]/TITCC nanoparticles after incubation in plasma.
- *Fig. 11*: shows UV-Vis spectra of PEI/Dye-12-aminododecanoic acid conjugate nanoparticles.

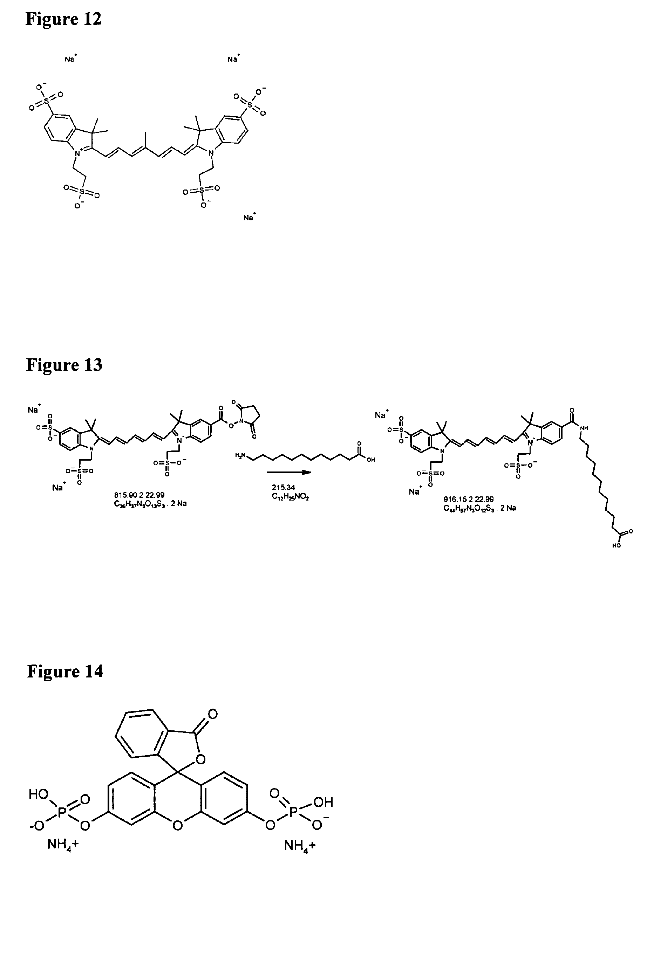

- *Fig. 12*: shows the chemical structure of trisodium-3,3-dimethyl-2-{4-methyl-7-[3,3-dimethyl-5-sulfonato-1-(2-sulfonatoethyl)-3H-indolium-2-yl]hepta-2,4,6-trien-1-ylidene}-1-(2-sulfonatoethyl)-2,3-dihydro-1H-indole-5-sulfonate, inner salt, abbreviated to TITCC.
- *Fig. 13*: shows the chemical equation of the synthesis of disodium 3,3-dimethyl-2-{7-[3,3-dimethyl-5- sulfonato-1-(2-sulfonatoethyl)-3H-indolium-2-yl]-hepta-2,4,6-trien-1-ylidene}-1-(2-sulfbnatoethyl)-2,3-dihydro-1H-indole-5-carboxylic acid-(11-carboxyundecyl)- amide, abbreviated to Dye-12-aminododecanoic acid conjugate.
- *Fig. 14*: shows the chemical structure of fluorescein diphosphate ammonium salt.

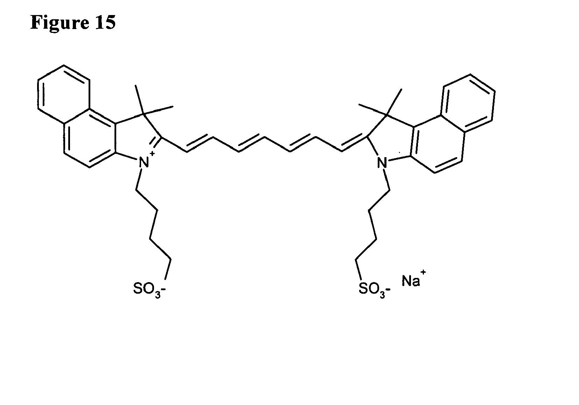

- *Fig. 15*: shows the chemical structure of indocyanine green.

### Examples

### Example 1: Optically fluorescent nanoparticles based on PEI

### (a) Preparation of PEI based nanoparticles

An aqueous solution of 0.1 % (w/v) PEI (1.8, 10, 70, or 750 kDa) is gently stirred, and an aqueous solution of 0.02 % (w/v) TITCC is instantly added. This composition is further agitated at about 4°C for about 30-45 minutes under UV protection. The aggregation progress is monitored by UV-Vis spectra starting from 900 nm down to 600 nm. TITCC dye has been shown to possess fluorescent activity when incorporated into PEI nanoparticles, and aggregate formation can be monitored on the basis of a shift of the UV-Vis spectrum (*Fig. 4*) The nanoparticle dispersion is concentrated by ultrafiltration and lyophilised after addition of a cryoprotector such as mannitol or lactose.

As shown in *Fig.* 4, the complete formation of dye molecules to J-aggregated had taken place in case of 150, 175, and 200 % surplus of PEI. This is confirmed by the absence of the starting wavelength of 756 nm of the pure dye, and by the new wavelength maximum at approximately 800 nm from the J-aggregates. In case of 100 and 125 % surplus of PEI, the two wavelength maxima indicate that the dye is only partially bound as J-aggregate. Therefore, the wavelength of the pure dye and the wavelength maximum of the J-aggregates can be detected side by side. All measured samples contained the same concentration of dye. The absorption is reduced due to the quenching effects in the J-aggregates. UV-Vis spectra were recorded by UV-2401-PC spectrophotometer (Shimadzu Corp.).

As shown in *Fig.* 5, the farthest wavelength shift from 756 nm to 810 nm is obtained with the smallest amount of surplus of cationic polymer. This may be due to a more compact dye-polymer-complex if fewer cationic charges for stabilisation are available. Vice versa, the complexes are less compact having a greater amount of polymer which results in a smaller shift to approximately 795 nm.

In case of a surplus of cationic polyelectrolyte, 100 % formation of J-aggregates is a result. Up to about 100-200 % of surplus, the nanoparticles had a smaller size due to an optimised electrostatical stabilisation. Vice versa, the closer the ratio of TITCC to PEI is to 1:1, the more the complex become destabilised with the consequence of precipitation and sedimentation of bigger particles. By adjusting the amount of PEI in relation to TITCC, the nanoparticle size can be varied in the range of 20-700 nm. Size was determined by DLS (Dynamic Light Scattering) with a "Zetasizer 3000" from Malvern Instruments. The preparation method via ionic self assembly resulted in particles with a narrow particle size distribution. Modified and unmodified particles *(Fig. 6)* showed a polydispersity index below 0.1. Zeta potential measurement were carried out under constant pH. As shown in *Fig. 6,* the nanoparticles had a constant size over two weeks with and without modification indicating their stability in aqueous solution.

The particles possess a spherical shape as shown by TEM observation (*Fig. 2*), and have a sized of approximately 100 nm. A spherical shape is a crucial factor for the determination of nanoparticles' size based on photocorrelation spectroscopy (PCS) with dynamic light scattering (DLS).

Furthermore, the surface potential of unmodified particles was determined as zeta potential of 45-55 mV, indicating that the cationic charges of the PEI chains form a stabilizing shell around the nanoparticle. PEI chains which remain uncomplexed cause an sterical and electrostatical stabilisation.

As shown in *Fig. 7*, the constantly lowered surface charge of modified nanoparticles confirms the successful surface modification with anionic compounds. Although the Zeta potential is close to 0 mV, these nanoparticles were stable in size as to be seen in *Fig. 6.* This is due to a combination of electrostatical and sterical stabilisation.

A scheme depicting the principle of co-aggregate formation according to the present invention is shown in *Fig. 1.*

### (b) Surface modification of PEI based nanoparticles

The nanoparticle's surface can be modified by taking advantage of electrostatic interactions. For that purpose, the block-co-polymer PEG[110]-b-GLU[10] can be used resulting in increased half-life of the nanoparticles in blood plasma as demonstrated *in vitro* experiments. Furthermore, the use of NADP disodium salt in addition to PEG[110]-GLU[10] results in enhanced fluorescence intensity.

A scheme depicting the principle of surface modification is shown in *Fig. 3.*

### (c) Nanoparticles based on PEG[113]-PEI[30]

Enhanced stability of the nanoparticles can be obtained using PEG[113]-PEI[30], probably due to the additional PEG block.

The progress of disaggregation is slowed-down compared to that with nanoparticles as described in Example 1. During disaggregation, a very sharp isosbestic point is observed indicating that the optically fluorescent agent exists in the two opposite states only (free/co-aggregated) without the presence of any intermediate states.

### Example 2: Nanoparticles based on protamine sulphate

Cationic protamine sulphate is widely used *in vivo* as heparin antagonist. Thus, nanoparticles comprising protamine sulfate as cationic polyelectrolyte are superior due to experienced less toxicity.

### Example 3: Nanoparticles based on P(DMAPMAM)

A solution of 0.1 % P(DMAPMAM) in water/acetone (10:1. by volume) is gently stirred while a solution of 0.02 % TITCC is added instantly. Under further agitation, formation of the aggregates takes place while acetone is removed.

### Example 4: Nanoparticles based on beta-cyclodextrin phosphate, PEI and ICG

An aqueous solution of 0.1 % beta-cyclodextrin phosphate is mixed with an aqueous solution of 0.02 % indocyanine green (ICG) and is further stirred for about 1 h. This mixture is injected into an aqueous solution of 0.1 % PEI (25 or 750 kDa). The aggregation progress is monitored by UV-Vis spectra starting form 900 nm down to 600 nm. The nanoparticle suspension is concentrated by ultrafiltration and lyophilized after addition of a cryoprotector.

### Example 5: Nanoparticles based on PEI/Dye-12-aminododecanoic acid conjugate and lauric acid

### (a) Preparation of the nanoparticles

For preparation of the nanoparticles, an aqueous solution of 0.01 % of polyethyleneimine, 25 kDa, and aqueous solution of 0.1 % of Dye-aminododecanoid acid conjugate (see below) and an ethanolic solution containing 1 % of lauric acid were prepared. The solution of the Dye-aminododecanoic acid conjugate was mixed with the lauric acid solution. This mixture was instantly added under constant stirring to the PEI solution which resulted in precipitation. The nanoparticle dispersion was stirred for removal of ethanol for ca. 24 h. The spectroscopic properties and the size can be varied by use of different charge ratios of the three compounds. A complex formation is also possible in the absence of pure lauric acid.

### (b) Preparation of the Dye-12-aminododecanoic acid conjugate

Synthesis of disodium 3,3-dimethyl-2-{7-[3,3-dimethyl-5-sulfonato-1-(2-sulfonatoethyl)-3H-indolium-2-yl]-hepta-2,4,6-trien-1-ylidene}-1-(2-sulfonatoethyl)-2,3-dihydro-1H-indole-5-carboxylic acid-(11-carboxyundecyl)amide = Dye-12-aminododecanoic acid conjugate (*Fig. 13:* chemical equation for the synthesis of Dye-12-aminododecanoic acid conjugate).
(1) Conversion of disodium 3,3-dimethyl-2-{7-[3,3-dimethyl-5-sulfonato-1-(2-sulfonatoethyl)-3H-indolium-2-yl]-hepta-2,4,6-trien-1-ylidene}-1-(2-sulfonatoethyl)-2,3-dihydro-1H-indole-5-carboxylic acid into the N-hydroxysuccinimidyl ester
   A mixture of 0.15 mg (0.2 mmol) dye, 0,23 g (2.0 mmol) N-hydroxysuccinimide in 8 mL of dimethylformamide is treated with a solution of 0.2 mg (1.0 mmol) N,N'-dicyclohexylcarbodiimide (DCC) in 3 ml of dimethylformamide and stirred for 4 h at room temperature. The mixture is poored into diethylether and the resulting solid collected by centrifugation. The process of precipitation from dimethylformamide using diethylether isrepeated 3-4 times. The NHS-ester is dried under nitrogen and directly used in the next step.
(2) Reaction with 12-aminododecanoic acid
   A solution of 15 mg (0.018 mmol) of the NHS-ester and 11 mg (0.05 mmol) of 12-aminododecanoic acid CAS[693-57-2] in 0.5 ml of dimethylformamide and 0.3 ml of dichloromethane is stirred for 36 h at 40 °C. After precipitation with diethylether, the resulting solid is dissolved in water/methanol and purified by preparative HPLC (RP 18, eluens water/methanol). The product is obtained as a blue lyophilisation product (yield 4.2 mg).

### Example 6: Properties of modified PEI/TITCC

As to be seen in *Fig.* 6, modified nanoparticles showed a slight particle size increase compared to unmodified nanoparticles which is due to the additional surface layer consisting of NADP disodium salt and PEG[110]-b-Glu[10]. Over a time period of 14 days, both modified and unmodified particles remained stable according to the constant particle size data and particle size distribution. From these data it can be concluded that a successful and permanent surface modification was obtained. Further evidence can be obtained from *Fig. 5* showing results from zeta potential measurements carried out under constant pH.

The surface potential was lowered by surface modification from about +40 to +45 mV down to about +5 mV (*Fig. 7*). The modifying layer consisting of NADP disodium salt and PEG[110]-b-Glu[10] provided a maximum of sterical stabilisation due to the PEG block. Therefore, the minimization of electrostatic stabilisation had no destabilising effect as can be concluded from size and surface charge constancy.

*Fig. 8* shows that nanoparticles prepared and modified according to Example 1 (a) and (b) remain stable up to 48 h when stressed at 37°C in an incubation shaker.

*Fig. 9* shows that the disaggregation of modified nanoparticles in blood plasma is nearly completed after about 15 h, wherein the half-life is about 3 h.

### Example 7: Nanoparticle stability in plasma

*Fig. 10* shows the disaggregation of PEG[113]-PEI[30]/TITCC nanoparticles after incubation in plasma. The nanoparticulate complexes in which the dye molecules are highly ordered as J-aggregates has an absorption maximum of 795 nm. When the nanoparticles disaggregate into their components, free dye and polyelectrolyte, the maximum shifts from 795 nm to 756 nm. All spectra meet one sharp isobestic point confirming that only two state of the dye occur, namely bound as J-aggregate within the nanoparticle and free after disaggregation. Furthermore, the absorption increases within the disaggreation process due to missing of quenching effects.

### Example 8: Measurement of the nanoparticles' size

The size of the nanoparticles was determined with a "Zetasizer 3000" ( Malvern Instruments) via the principle of PCS (Photon Correlation Spectroscopy) based on DLS (dynamic light scattering). In addition, the size was determined by TEM (transmission electron microscopy). The images taken confirmed the spherical shape of the nanoparticles which is a crucial factor for size measurement via the PCS method. Measurements were conducted with appropriate diluted samples at constant temperature ( 25°C) and at a defined viscosity of the solution.

PCS is an appropriate method for size determination of particles having a diameter from 3 nm to 3 µm. The molecules of the solvent are in a permanent movement, driven by the Brownian motion. This results in non-directional movements of the particles after their collision with the solvent molecules. The smaller the size of the particles, the faster their movements. If a laser light beam is focused on a sample of particles, the light is scattered on the particles' surface. The intensity of the scattered light fluctuates due to the non-directional movements of the particles and as a function of time. The smallest and therefore fastest moving particles cause the highest fluctuations of the intensity of the scatterred light. Under an angle of 90° these fluctuations are detected. By means of these fluctuations, one can determine the size distribution on the base of an autocorrelation function. The mean hydrodynamic diameter is calculated from the gradient of the correlation function.

### Example 9: Measurement of the nanoparticles' surface charge

Surface charges of the particles were determined as zeta potential with a "Zetasizer 3000" (Malvern Instruments).

Equal concentrations of the nanoparticle dispersion were diluted with MilliQ water and measurements were performed at constant pH and constant temperature. The zeta potential measurement is based on the principle of LDA (Laser Doppler Anemometry). The zeta potential is the potential along the shearing surface of a moving particle if the largest portion of the diffuse layer has been sheared by the movement of this particle.

Particles with a charged surface move in an electrical field to the oppositely charged electrode and the velocity of the moving particles depends on the amount of surface charges and the electric field strength. The electrophoretic mobility is the ratio of the velocity and the electric field strength. The product of electrophoretic mobility and factor 13 provides the zeta potential [mV].

The velocity of the particles in the electric field is determined on the principle of LDA. Therefore, a laser beam is focused on the particles, which are moving within the electrical field and the scattered laser light is detected. Due to the movement of the particles, a shift of the reflected laser wavelength compared to the primary laser wavelength is observed. The magnitude of the frequency shift depends on the velocity and is called Doppler frequency shift (Doppler effect). By means of the Doppler frequency shift, the scattering angle and the wavelength the velocity can be determined.

### References

Bellocq, N.C., Pun, S.H., Jensen, G.S., Davis, M.E. (2003), Transferrin-Containing, Cyclodextrin Polymer-Based Particles for Tumor-Targeted Gene Delivery, Bioconjug Chem. 14: 1122-1132.
Boudad, H., Legrand, P., Lebas, G., Cheron, M., Duchene, D., Ponchel, G. (2001), Combined hydroypropyl-beta-cyclodextrin and poly(alkylcyanoacrylate) nanoparticles intended for oral administration of squinavir; Int J Pharm 62: 263-268.
Cavalli, R., Peira, E.,Caputo, O., Gasco, M.R. (1999) Solid lipid nanoparticles as carriers of hydrocortisone and progesterone complexes with beta-cyclodextrin; Int J Pharm 182:59-69.
Dass, C.R. et al. (2000), Drug. Deliv 7: 15-20.
Derycke, A.S., P.A.M. Witte (2004), Liposome's for photodynamic therapy; Adv Drug Deliv Rev 56: 17-30.
Duchene, D., Wouessidjewe, D., Ponchel, G. (1999), Cyclodextrins and carrier systems; J Control Release 62: 263-268.
Intes, X., B. Chance (2005), Non-PET functional imaging techniques: optical; Radiol Clin North Am 43: 221-234.
Jelly, E.E. et al. (1936) Nature 138: 1009-1010.
Josephson, L., M.F. Kirchner, U. Mahmood,Y. Tang, R. Weissleder(2002), Near-Infrared Fluorescent Nanoparticles as Combined MR/Optical Imaging Probes; Bioconjug Chem 13: 554-560.
Kobayashi, T. (1996), World Scientific Publishing Co. Pte. Ltd.
Liotta, L.A. et al. (1976), Cancer Res 36: 889-894.
Pun, S.H., Tack, F., Bellocq, N.C., Cheng, J., Grubbs, B.H., Jensen, G.S., Davis, M.E., Brewster, M., Janicot, M., Janssens, B., Floren, W., Bakker, A. (2004), Targeted delivery of RNA cleaving DNA Enzyme to Tumor Tissue by Transferrin-Modified, Cyclodextrin-Based Particles; Cancer Bio Therapy 3: 641-650.
Saxena, V., M. Sadoqi, J. Shao (2004a); Enhanced photo-stability, thermal stability and aqueous-stability of indocyanine green in polymeric nanoparticulate systems, J Photochem Photobiol B: 74: 29-38.
Saxena, V., M.Sadoqi, J. Shao (2004b), Indocyanine green-loaded biodegradable nanoparticles: preparation, physicochemical characterization and in vitro release, Int J Pharm 278: 293-301.
Rousseau et al. (2002), Photochem Photobiol Sci 1: 395-406.
Tung, C.,Y. Lin, W.K. Moon, R. Weissleder (2002), A Receptor-Targeted Near Infrared Fluorescence Probe for In Vivo Tumor Imaging, Chembiochem 8: 784-786.
R. Weissleder (2001 a), A clearer vision for in vivo imaging, Nat Biotechnol 19: 316-317.
R. Weissleder (2001b), Molecular imaging (special review), Radiology 219: 316-333.

## Claims

1. A nanoparticle matrix comprising a co-aggregate of at least one charged polyelectrolyte and at least one oppositely charged active agent, wherein the active agent is a hydrophilic optically fluorescent agent.

2. The nanoparticle matrix according to claim 1, wherein the ratio of total polyelectrolyte charge and total optically fluorescent agent charge results in a charge surplus.

3. The nanoparticle matrix according to claim 1 or 2, wherein the optically fluorescent agent is a visible- or NIR-emissive compound.

4. The nanoparticle matrix according to any of the preceding claims, wherein the optically fluorescent agent is a NIR-emissive compound.

5. The nanoparticle matrix according to any of the preceding claims, wherein the optically fluorescent agent comprises a planar aromatic and highly conjugated system.

6. The nanoparticle matrix according to any of the preceding claims, wherein the optically fluorescent agent shows a bathochromic shift in UV-Vis absorption spectrum during co-aggregate formation.

7. The nanoparticle matrix according to claim 6, wherein the optically fluorescent agent shows a hypsochromic shift in UV-Vis absorption spectrum during disaggregation.

8. The nanoparticle matrix according to claim 6 or 7, wherein the bathochromic and/or hypsochromic shift is **characterised by** a Δ_{wavelength} in the range of about 20 nm to about 80 nm.

9. The nanoparticle matrix according to any of claim 6 to 8, wherein the bathochromic and/or hypsochromic shift is **characterised by** a Δ_{wavelength} in the range of about 40 nm to about 50 nm.

10. The nanoparticle matrix according to any of claims 6 to 9, wherein the bathochromic and/or hypsochromic shift is **characterised by** a Δ_{wavelength} of about 44 nm.

11. The nanoparticle matrix according to any of the preceding claims, wherein the optically fluorescent agent shows a decrease in absorption intensity during co-aggregate formation.

12. The nanoparticle matrix according to claim 11, wherein the optically fluorescent agent shows an increase in absorption intensity during disaggregation.

13. The nanoparticle matrix according to any of the preceding claims, wherein the polyelectrolyte is cationic and the optically fluorescent agent is anionic.

14. The nanoparticle matrix according to claim 13, wherein the ratio of total polyelectrolyte charge and total optically fluorescent agent charge is in the range of about 1.5:1 to about 6:1.

15. The nanoparticle matrix according to claim 14, wherein the ratio of total polyelectrolyte charge and total optically fluorescent agent charge is in the range of about 1.5:1 to about 3:1.

16. The nanoparticle matrix according to claim 15, wherein the ratio of total polyelectrolyte charge and total optically fluorescent agent charge is about 1.5:1.

17. The nanoparticle matrix according to any of claims 13 to 16, wherein the cationic and polyelectrolyte is selected from the group consisting of polyethyleneimine and derivatives such as PEG[113]-b-PEI[30]; polyvinyl derivatives such as polyvinylamine polyvinylpyridin; polyarginine, polyhistidine; polylysine, lysine octadecyl ester; polyguanidine and poly(methylene-co-guanidine); protamines such as protamine sulfate; polyallylamine, polydiallyldimethylamine; polymethacrylates such as Eudragit E, poly(dimethyl-aminopropyl-methacrylamide) [P(DMAPMAM)] or poly(dimethylaminoethyl-methacrylate) [P(DMAEMA]; spermine, spermidine and their polymers polyspermine and polyspermidine; polyhistidine; quaternised polyamide, poly(dimethyl-aminoethyl-aspartamid) [PDAA]; modified silicones; polyphosphazene; proteins such as histones; modified starch, modified gelatine, modified cellulose such as aminated celluloseethers; aminated dextrans, aminated cyclodextrins, aminated pectines; chitosan; and salts and derivatives thereof.

18. The nanoparticle matrix according to claim 17, wherein the polyelectrolyte is polyethyleneimine.

19. The nanoparticle matrix according to any of claims 13 to 18, wherein the optically fluorescent agent comprises a surplus of negatively charged groups selected from the group consisting of sulfonate and phosphate.

20. The nanoparticle matrix according to claim 19, wherein the optically fluorescent agent is a polymethine dye, preferably a cyanine dye.

21. The nanoparticle matrix according to claim 20, wherein the optically fluorescent agent is an indotricarbocyanine dye or an indodicarbocyanine dye.

22. The nanoparticle matrix according to claim 21, wherein the optically fluorescent agent is a tetrasulfonated indotricarbocyanine dye.

23. The nanoparticle matrix according to any of claims 19 to 22, wherein the optically fluorescent agent is trisodium-3,3-dimethyl-2-{4-methyl-7-[3,3-dimethyl-5-sulfonato-1-(2-sulfonatoethyl)-3H-indolium-2-yl]hepta-2,4,6-trien-1-ylidene}-1-(2-sulfonato-ethyl)-2,3-dihydro-1H-indole-5-sulfonate, inner salt, abbreviated to TITCC.

24. The nanoparticle matrix according to any of claims 19 to 22, wherein the optically fluorescent agent is disodium 3,3-dimethyl-2-{7-[3,3-dimethyl-5-sulfonato-1-(2-sulfonatoethyl)-3H-indolium-2-yl]-hepta-2,4,6-trien-1-ylidene}-1-(2-sulfonatoethyl)-2,3-dihydro-1H-indole-5-carboxylic acid-(11-carboxyundecyl)-amide, abbreviated to Dye-12-aminododecanoic acid conjugate.

25. The nanoparticle matrix according to any of the preceding claims, wherein the matrix additionally comprises at least one auxiliary electrolyte.

26. The nanoparticle matrix according to claim 25, wherein the auxiliary electrolyte is selected from the group consisting of modified cyclodextrins, chelating agents, dendrimers and crown ethers.

27. The nanoparticle matrix according to claims 25 or 26, wherein the auxiliary electrolyte is charge opposite to the polyelectrolyte charge.

28. The nanoparticle matrix according to any of claims 26 or 27, wherein the modified cyclodextrin is an anionic cyclodextrin, selected from the group consisting of phosphated, sulfated, carboxymethylated and succinylated cyclodextrin.

29. The nanoparticle matrix according to any of claims 26 to 28, wherein the anionic cyclodextrin is heptakis-(2,3-dimethyl-6-sufato)-beta-cyclodextrin or heptakis-(2,6-diacetyl-6-sulfato)-beta-cyclodextrin.

30. The nanoparticle matrix according to any of claims 26 to 28, wherein the anionic cyclodextrin is beta-cyclodextrin phosphate.

31. A nanoparticle comprising the nanoparticle matrix according to any of claims 1 to 30.

32. The nanoparticle according to claim 31, wherein the nanoparticle is non-vesicular.

33. The nanoparticle according to claim 31 or 32, wherein the nanoparticle size is in the range of 10 nm to 1.2 µm.

34. The nanoparticle according to claim 33, wherein the nanoparticle size is in the range of 10 nm to 500 nm.

35. The nanoparticle according to claim 34, wherein the nanoparticle size is in the range of 10 nm to 300 nm.

36. The nanoparticle according to any of claims 31 to 35, wherein the nanoparticle comprises at least one surface modifying agent.

37. The nanoparticle according to claim 36, wherein the surface modifying agent is charged opposite to the nanoparticle's surface charge.

38. The nanoparticle according to claims 36 or 37, wherein the surface modifying agent is covalently or non-covalently bound to the nanoparticle's surface.

39. The nanoparticle according to any of claims 36 to 38, wherein the surface modifying agent is PEG[110]-GLU[10].

40. The nanoparticle according to any of claims 36 or 39, wherein the surface modifying agent is selected from the group consisting of NADP, AMP, cAMP and ADP and salts thereof.

41. The nanoparticle according to any of claims 31 to 40, wherein the nanoparticle comprises a targeting structure.

42. The nanoparticle according to claim 41, wherein the targeting structure is a passively targeting structure.

43. The nanoparticle according to claim 41, wherein the targeting structure is an actively targeting structure.

44. The nanoparticle according to claim 43, wherein the actively targeting structure is a region of an antibody, of a non-antibody ligand, of an aptamer, or of fragments thereof.

45. The nanoparticle according to any of claims 31 to 44, wherein the nanoparticle comprises a therapeutically active agent.

46. The nanoparticle according to claim 45, wherein the therapeutically active agent is selected from the group consisting of anti-proliferating agents, anti-inflammatory agents, and dyes for photodynamic therapy.

47. A method of preparation of a nanoparticle according to any of claims 31 to 46 comprising the following steps:
(a) contacting at least one polyelectrolyte and at least one optically fluorescent agent;
(b) co-aggregating the polyelectrolyte and the optically fluorescent agent under UV protection;
(c) yielding the co-aggregation product.

48. The method according to claim 47, additionally comprising the following step:
(b') modifying the co-aggregation product's surface.

49. The method according to claim 47 or 48, wherein the co-aggregation in step (b) is carried out at 4°C.

50. The method according to any of claims 47 to 49, wherein the co-aggregation in step (b) is carried out at pH 7.0-9.0.

51. The method according to claim 50, wherein the co-aggregation in step (b) is carried out at pH 7.5-8.5.

52. The method according to any of claims 47 to 51, wherein the co-aggregation in step (b) is monitored by running an UV-Vis spectrum.

53. A method of surface modification of a nanoparticle prepared according to any of claims 47 to 52 comprising the step of applying a surface modifying agent according to any of claims 36 to 40 onto the nanoparticle's surface.

54. A use of a nanoparticle according to any of claims 31 to 46 for *in vitro* application.

55. A method for *in vitro* diagnosis using nanoparticles according to any of claims 31 to 46 comprising the following steps:
(a) contacting the nanoparticle with a biological sample;
(b) removing non-bound nanoparticles from the biological sample;
(c) detecting nanoparticles bound to the biological sample;
(d) comparing the result obtained in step (c) with a result obtained from a control sample.

56. The method according to claim 55, wherein the detection in step (c) is carried out by using a fluorescent microscopic technique.

57. A use of a nanoparticle according to any of claims 31 to 46 for *in vivo* application.

58. A method for *in vivo* diagnosis using nanoparticles according to any of claims 31 to 46 comprising the following steps:
(a) applying the nanoparticle to a subject;
(b) detecting nanoparticles accumulated in the subject.

59. The method according to claim 58, wherein the detection in step (b) is carried out by using a CCD technique.

60. A method for *in vivo* localization of a nanoparticle according to any of claims 31 to 46.

61. The method according to claim 60, wherein the nanoparticle comprises at least one therapeutically active agent according to claims 45 or 46.

62. A use of a nanoparticle according to any of claims 31 to 46 for the manufacture of a pharmaceutically acceptable composition.

63. A pharmaceutically acceptable composition comprising a nanoparticle according to any of claims 31 to 46.

64. A kit for *in vitro* and/or *in vivo* diagnosis comprising a nanoparticle according to any of claims 31 to 46.
